# EUROPEAN PATENT SPECIFICATION

(11) **EP 3 638 597 B1**
(45) Date of publication and mention of the grant of the patent: **04.01.2023**
(21) Application number: 18740319.1
(22) Date of filing: 12.06.2018
(51) Int. Cl.: B65D 21/02, A01K 67/033, B65D 85/50

(54) **CONTAINER AND COMBINATION OF CONTAINER AND SPAWN STRUCTURE**
BEHÄLTER UND KOMBINATION AUS BEHÄLTER UND BRUTSTRUKTUR
CONTENANT ET COMBINAISON DE CONTENANT ET DE STRUCTURE DE FRAI

(30) Priority: 15.06.2017 NL 2019079
(43) Date of publication of application: 22.04.2020
(60) Divisional application: 22208475.8
(73) Proprietor: Ynsect NL R & D B.V., 3852AB Ermelo (NL)
(72) Inventor: STAAL, Simon, 2645 NK Delfgaum (NL); BOLIER, Lucas Jan, 25120 ED Den Haag (NL); DE BRUIN, Johan, 3882 RE Putten (NL)
(74) Representative: Santarelli
(86) International application number: PCT/NL2018/050380
(87) International publication number: WO 2018/231053

(56) References cited:
- EP-A- 0 092 888
- WO-A2-2016/153340
- BE-A- 697 232
- FR-A- 1 240 706
- FR-A1- 2 703 027
- NL-A- 7 216 496

## Description

The first aspect of the invention relates to an open topped, stackable, molded plastic container, which is generally rectangular with a bottom and first and second pairs of parallel upright walls joined to the bottom and at corners via corner structures.

From the prior art, stackable containers are well known. To be suitable for the purpose of breeding and rearing insects, a type of containers is desired which is stackable up to a height of at least 15 containers, and which can be used in a climate housing, having a temperature up to 40°C and a humidity of up to 90%.

Stackable plastic containers with corners structures are e.g. known from EP0092888, which discloses a container according to the preamble of claim 1, and NL7216496. FR1240706 describes another stackable plastic container having triangular hollow corner structures, including short tube-shaped feet which protrude below the container bottom, for inserting into holes on top of corresponding stackable containers. FR2703027 discloses stackable containers having load-bearing hollow corner posts. The lower ends of said posts end in short studs above the bottom plane of the container, for inserting into hollow corner posts of stacked containers.

WO2016/153340 of the same applicant discloses an insect spawning container to be used in an insect breeding facility for breeding insects fo the type with crawling mother beetles having a protractable egg-laying tube, such as lesser mealworms or zophobas morios, in which insect spawning container at least one spawn structure is provided in which the mother insects will spawn their eggs, wherein the insect spawning container comprises a bottom, and the at least one spawn structure adjoins the bottom, the at least one spawn structure having a scalable face such that the mother beetles can crawl from the bottom onto and up along the scalable face of the spawn structure, and the at least one spawn structure comprising a multitude of crevices accessible from the scalable face, the crevices having dimensions tuned to the egg-laying tube of the mother beetles.

According to the present invention, the corner structure of the container comprises:
- a substantially diagonal and upright wall section which, seen in top view, extends diagonally between adjoining upright walls and blends into said upright walls to define the contour of a containment space of the container; and
- a top wall extending outward from an upper end of the diagonal and upright wall section; and
- a hollow tubular post extending vertically downward from said top wall, said hollow tubular post being spaced from said diagonal and upright wall section, wherein the top wall has a hole aligned with the hollow tubular post;
- wherein multiple, e.g. at least three, vertical stacking ribs are arranged circumferentially spaced about the hollow tubular post, each stacking rib having a vertical inner end joined to said hollow tubular post,
   ∘ wherein at least one, preferably two, of said stacking ribs have a vertical outer end joined to said diagonal and upright wall section and/or to said adjoining upright wall section; and
   ∘ wherein each of said stacking ribs has a lower end, said lower ends being located in a common plane;
wherein said tubular post has a protruding stacking pin portion extending downward beyond said common plane of the stacking rib lower ends; such that - in a stack - the pin portion of an upper container is received through the hole in the top wall of the corner structure of a lower container onto which said upper container is stacked.

This type of corner structure provides structural stability to stacks of at least 21 containers on top of each other, in combination with a relatively large containment space.

The open topped, stackable, plastic container is preferably made from polypropylene (PP), and is preferably monolithically injection molded.

In a stack is the pin portion of an upper container received through the hole in the top wall of the corner structure of a lower container onto which said upper container is stacked. The stacking rib lower ends of the upper container, located in a common plane, rest onto the top wall, adjacent the hole, of the lower container.

In embodiments, said stacking pin portion does not protrude downward beyond the bottom of the container (or terminates above the bottom) and the lower ends of the stacking ribs lie above the bottom. As a result, prior to stacking, a container rests on the floor with its bottom, and in a stack, the upper container nests in the lower container. Once stacked, the top wall of the lower container is adjacent the first and second pairs of parallel upright walls of the upper container.

In embodiments wherein the stacking pin does protrude downward beyond the bottom of the container, the container, prior to stacking, will rest onto these stacking pins. In embodiments with the lower ends of the stacking ribs above the bottom, the upper container will nest in the lower container. In embodiments wherein the lower ends of the stacking ribs lie below the bottom, in a stack, the upper container does not nest in the lower container but will be located at a distance above this lower container.

Advantageously, the upper ends of said stacking ribs join said top wall. This is in particular advantageous in view production of the container by injection moulding. The stacking ribs have an essentially rectangular cross-section. Also advantageous in view of the production is to design the rigs tapering in the direction of the stacking pin, e.g. to a thickness at the lower ends of 3,2 mm.

In embodiments, the corner structure further comprises quarter-circular skirt walls, extending (depending) downwards from an outward perimeter of the top wall. Preferably at least one of said stacking ribs has an has an outer vertical end joined which is not joined to said diagonal and upright wall section and/or said adjoining upright wall section, but to said to said quarter-circular skirt wall.

Hence, the inner ends of each stacking rib are joined to the post, and preferably the upper ends of said stacking ribs join the top wall. At least one, preferably two, of said stacking ribs have an outer vertical end joined to said diagonal and upright wall section and/or said adjoining upright wall section. Possibly, one or more other stacking ribs have an outer vertical end joined to a quarter-circular skirt wall.

In embodiments, the bottom of the spawning container is provided with spawn structure clamps, to be used in combination with a spawn structure according to the second aspect of the present invention.

In embodiments, top flanges extend outward from an upper end of the upright walls. Advantageously, these top flanges and/ or the top walls are provided with one or more drainage holes. It is conceivable that the top flanges extend parallel to the bottom of the container, but it is also possible that parts of the upright wall are sloped, resulting in top flanges extending at an angle with respect to the bottom.

Advantageously, flange skirt walls depend downwards from the top flanges, essentially parallel to and at a distance from the respective first and second pairs of parallel upright walls. The skirt walls of top flanges extending parallel to the bottom and extending at an angle with respect to the bottom may blend into each other. Advantageously, the top flanges have a common width, preferably corresponding to the width of the top wall adjacent the upright walls. Preferably, at least one of the skirt walls is provided with a label, e.g. via in-mould labelling.

The invention also relates to the use of such a container in an insect breeding facility, e.g. for breeding and/ or rearing insects. Furthermore, the first aspect relates to a stack comprising at least 15, preferably at least 20 containers. Such a container may have a content weight of 15-20kg.

Further, the invention relates to a climate housing of an insect breeding facility, housing multiple stacks of inventive containers.

A second aspect, not part of the invention relates to the combination of an insect spawning container and at least one spawn structure. Such a combination is known from WO2016/153340 of the same applicant, disclosing an insect spawning container to be used in an insect breeding facility for breeding insects of the type with crawling mother beetles having a protractable egg-laying tube, such as lesser mealworms or zophobas morios, in which insect spawning container at least one spawn structure is provided in which the mother insects will spawn their eggs, wherein the insect spawning container comprises a bottom, and the at least one spawn structure adjoins the bottom, the at least one spawn structure having a scalable face such that the mother beetles can crawl from the bottom onto and up along the scalable face of the spawn structure, and the at least one spawn structure comprising a multitude of crevices accessible from the scalable face, the crevices having dimensions tuned to the egg-laying tube of the mother beetles.

The aim of the second aspect is to provide an improved combination, providing an increased yield in an insect breeding process.

This is achieved by providing a combination comprising:
- an insect spawning container adapted for use in an insect breeding facility for breeding insects of the type with crawling mother beetles having a protractable egg-laying tube, such as lesser mealworms or zophobas morios; wherein the insect spawning container comprises a bottom and first and second pairs of parallel upright walls;
- at least one spawn structure in which the mother insects will spawn their eggs, comprising a foldable member with a fold section provided centrally between, and via hinges connected to, a perforated left-hand section and a perforated right-hand section; the spawn structure further comprising a rigid plate;

wherein the foldable member and the rigid plate are movable with respect to each other between an open configuration and a closed spawning configuration;
wherein in the closed spawning configuration the foldable member is folded such that the left-hand section and the right-hand section are parallel to each other, with the rigid plate sandwiched between the sections, creating a multitude of crevices between the rigid plate and inner faces of the folded member, the crevices having dimensions tuned to the egg-laying tube of the mother beetles; and in which spawning configuration outer faces of the folded member form two climbing faces;
wherein the spawn structure in the closed spawning configuration is positioned in the container such that the mother beetles are able to crawl from the bottom onto and up along the climbing faces of the spawn structure into the crevices;
and wherein in the open configuration the foldable member is folded open allowing harvesting of the eggs.

This is an advantageous configuration as folding a foldable member around the rigid plate allows the opening by a sideways movement of the foldable member, similar to the opening of a book, diminishing the risk of damaging the eggs, as no transversal relative sliding movement of the foldable member and rigid plate of the spawn structure is possible. Furthermore, the dimensions of the crevices are well-defined and reproducible. As a result, the yield is increased.

The second aspect further relates to a method for breeding insects of the type with crawling mother beetles having a protractable egg-laying tube, such as lesser mealworms or zophobas morios, comprising the steps of:
- providing a plurality of spawning containers in combination with spawn structures in the closed spawning configuration, as described above;
- providing adult insects including mother insects to the spawning containers; and periodically repeating the steps of:
- removing a spawn structure from the spawning container, leaving the adult insects in the spawning container;
- folding open the foldable member of the spawn structure;
- removing the foldable member and/ or the rigid plate of the spawn structure holding the eggs, and allowing the eggs to hatch;
- providing an empty spawn structure in the closed spawning configuration in each spawning container.

The second aspect further relates to the use of the inventive combination.

The crevices in the spawning configuration of the spawn structure have dimensions allowing the entry and passage of a protracted egg-laying tube of the beetle into the crevice, to deposit her eggs in the crevices onto the foldable member and/or rigid plate of the spawn structures during spawning, while prohibiting the entry of the mouth into the crevice, and thus preventing the beetles to eat the eggs.

The deposition of eggs involves positioning the egg-laying tube into the crevice, and sticking the eggs onto a part of the spawn structure. This can be both the foldable member and rigid plate of the spawn structure. In embodiments, one of the foldable member and rigid plate is made non-sticking, ensuring that all the eggs stick onto the other part of the spawn structure. Advantageously, the foldable member of the spawn structure is non- sticking, allowing all eggs to be spawned onto the rigid plate of the spawn structure.

In an open configuration the distance between the foldable member and rigid plate of the spawn structure is increased. In the open configuration, an enlarged area (free space) around the eggs is created, which is advantageous to optimize the hatching climate. The eggs are quite vulnerable and when hatching out, the baby larvae need more space to get out of the crevices. When the crevices are too small, risks are that the eggs become moist, and that the larvae stick to the walls of the crevice. It is conceivable that the enlarged area is created at the start of the hatching process, or only at the end after several days of hatching, or therebetween.

The enlarged area may also only be obtained for the purpose of harvesting the baby larvae. It is also conceivable that the enlarged area is obtained only for the purpose of cleaning the spawn structure.

Advantageously, the entire spawn structure is removed from the spawning container, and in a subsequent step the distance between the parts of the spawn structure is increased, allowing the foldable member and/or rigid plate of the spawn structure comprising the eggs to hatch, e.g. to be transported to the hatch area. In embodiments where one of the parts of the spawn structure is made non- sticking, allowing all eggs to be spawned onto the other part of the spawn structure, it is advantageous to allow the part of the spawn structure with the eggs sticking onto it to hatch, e.g. in a hatching chamber, while cleaning the other part of the spawn structure. After cleaning, this other part can be assembled with a new part without eggs to form a closed spawning configuration to be placed into the spawning container. Advantageously, the eggs stick onto the rigid plate of the spawn structure. Hence, while allowing a rigid plate with eggs to hatch, another rigid plate can be placed in the cleaned foldable member and positioned back into the spawning container.

Advantageously, the foldable member has a length in the direction of the fold section, essentially corresponding to the length of the rigid plate, and wherein the width of the left-hand and right-hand sections essentially correspond to the width of the rigid plate. With such tuned dimensions, in the spawning configuration the foldable member and rigid plate match onto each other allowing handling of the spawn structure as a single entity. Furthermore, such a configuration allows optimization of the amount of crevices between the foldable member and rigid plate in a single spawn structure.

The rigid plate of the spawn structure advantageously has a length which is four times larger than its width. Accordingly, the foldable member of the spawn structure, prior to folding, has a length in the direction of the fold section that is roughly twice the width. For example, the length of the rigid plate of the spawn structure is 40-80 cm, and the height 10-20 mm. An advantageous thickness of the left-hand and right-hand sections of the foldable plate, as well as the rigid plate, is 2-5, preferably 3 mm.

The fold section has a width in the unfolded position in a range between a width essentially corresponding to the thickness of the second plate, and a width which is 1,1 - 2,5 times the thickness of the second plate.

The width of the fold section is e.g. dependent on the type of hinge. Advantageously, the foldable member of the spawn structure is provided with living hinges between the fold section on the one hand, and the left-hand and right-hand sections on the other hand. A living hinge is a thin flexible, or a so-called flexure bearing. The thickness of the foldable member at the location of such a living hinge may be reduced to less than 0,5, in particular 0,35mm. With such a living hinge, it is possible to have a width of the fold section essentially corresponding to the thickness of the second plate.

In embodiments, the fold section comprises a slit seat having a length corresponding to the length of the rigid plate, a width essentially corresponding to the thickness of the rigid plate, and a depth to accommodate the rigid plate. The depth of the slit is e.g. 5-15 mm, preferably around 10 mm. Advantageously, the width of the slit is tuned to the thickness of the rigid plate such that the rigid plate is clasped into the slit. In embodiments, the upper edges of the slit are provided with tilted guide surfaces to assist in guiding the rigid plate into the slit. With such a fold section comprising a slit, the fold section has a width in the unfolded position of about 2 times the thickness of the second plate. Also in such embodiments wherein the fold section comprises a slit, it is possible to provide a living hinge between the fold section and the left-hand and right-hand sections.

In embodiments, at least one of the hinges between the sections and the fold section is pre-stressed opposite the folding direction. As a consequence, upon folding the foldable member around the rigid plate of the spawn structure, the pre-stress has to be overcome to bring the left-hand section, the rigid plate and the right-hand section parallel to each other. This is in particular advantageous when the spawn structure is to be opened. With the eggs deposited onto the foldable member and/ or rigid plate of the spawn structure, the parts of the spawn structure may stick onto each other. With the pre-stressed hinge, the pre-stress assists in increasing the distance between the foldable member and rigid plate of the spawn structure, and overcoming the possible sticking force.

In embodiments, the ends of the inner faces of the foldable member opposite the fold section taper towards the outer faces. Possibly, instead thereof or in addition thereto the sides of the foldable member adjacent the fold section taper towards the outer faces. By allowing the ends to taper towards the outer faces, in the spawning configuration an opening slit is created, between the parallel left-hand section and the right-hand section on the one hand, and the rigid plate on the other hand. This opening slit facilitates the increasing of the distance between the foldable member and rigid plate of the spawn structure to the open configuration. Possibly, the opening slits allow the use of an opening tool to increase the mutual distance.

In embodiments, the bottom of the insect spawning container is provided with spawn structure clamps to receive the spawn structure in the spawning configuration and allow the spawn structure to be connected to the container.

Possibly, a spawn structure clamp comprises opposed protruding fingers, protruding from the bottom of the insect spawning container, between which a spawn structure receiving groove is provided. Possibly, the configuration of the opposed fingers is such that the bottom of the groove between the fingers is provided at a distance from the bottom of the spawning container, e.g. 2-4, preferably 3 mm. Advantageously, the fingers have tapering ends, to provide a guide surface for the spawn structure.

Preferably, the ends of the outer faces of the foldable member opposite the fold section are provided with complementary container connectors, allowing the spawn structure to be connected to the container in the spawning configuration. Advantageously, the configuration of the spawn structure clamps and the complementary container connectors is such that by connecting the spawn structure to the container, the position of the spawn structure in the container is defined. Possible container connectors are snap-fitments, protruding from the outer faces.

The spawn structure in the closed spawning configuration is positioned in the container such that the mother beetles are able to crawl from the bottom onto and up along the climbing faces of the spawn structure into the crevices. Possibly, the spawn structure is positioned adjoining the bottom of the container. It is also possible that the mother beetles crawl onto the climbing faces via the spawn structure clamps of the container.

In embodiments, the length of the spawn structure is between 70-100%, in particular 80-90%, of that of the insect spawning container, and wherein preferably 2-10, in particular 4-6 spawn structure are provided in the longitudinal direction of the spawning container. With multiple of such elongated spawn structures in the spawning container, an optimum number of eggs is harvested. Advantageously, the spawn structures are provided along the sides of the spawning container, leaving a clear central longitudinal space in the spawning container. In this clear space, having a width of essentially 30-70%, in particular 40-60% of the width of the spawning container, the mother beetles are free to move around, possibly receive food, and provide ventilation. Optionally, the bottom of the spawning container is in this clear space provided with a perforated area to allow the removal of excrements.

In the closed spawning configuration the foldable member and rigid plate of the spawn structures are positioned with respect to each other such that a multitude of crevices is created therebetween. The foldable member is provided with perforated sections, i.e. sections provided with cut-aways, wherein crevices are at least created between an outer contour of the perforation and the rigid plate of the spawn structure.

For breeding lesser mealworms the width (w) of the crevices is 0,2-1 ,2 mm, while for breeding zophobas morios the width of the crevices is 0,2-1 ,8 mm. The depth (d) of the crevices may vary between 0,5-10,0 mm.

For a spawn structure having a length of 40-80 cm, and a height 10-20 mm, advantageously the left-hand section and the right-hand section are each provided with 250-400 perforations, in particular 300-350.

The invention is further elucidated in relation to the drawings, in which:
Fig. 1a represents a side view of a stack of two containers according to the present invention;
Fig. 1b represents a perspective top view of a container according to the invention;
Fig. 2a represents a perspective bottom view of a corner structure of the container of figs. 1a and 1b;
Fig. 2b represents a detailed side view of a corner structure of the container of figs. 1a and 1b;
Fig. 2c represents a cross-section of the corner structures of two stacked containers of fig. 1a;
Fig. 2d represents a bottom view of the corners structure of the container of figs. 1a and 1b;
Fig. 3a-3e represent a foldable member of a spawn structure of a combination according to a second aspect not forming part of the invention, respectively in a perspective view from below, a top view, a side view, a perspective view from above and a detail thereof;
Fig. 4 represents a perspective view onto a spawn structure according to a second aspect not forming part of the invention in the open configuration;
Figs. 5a and 5b represent perspective views onto the entire spawn structure according to a second aspect not forming part of the invention in the closed configuration and a detail thereof;
Figs. 6a-6d represent details of the fold section of the foldable member of the spawn structure according to a second aspect not forming part of the invention;
Figs. 7a, 7b and 8a-8d represent detailed perspective views of a spawn structure clamp and a complementary container connector on the foldable plate;
Figs. 9a and 9b represent a perspective view and a top view of a combination of an inspect spawning container and a spawn structure according to the second aspect

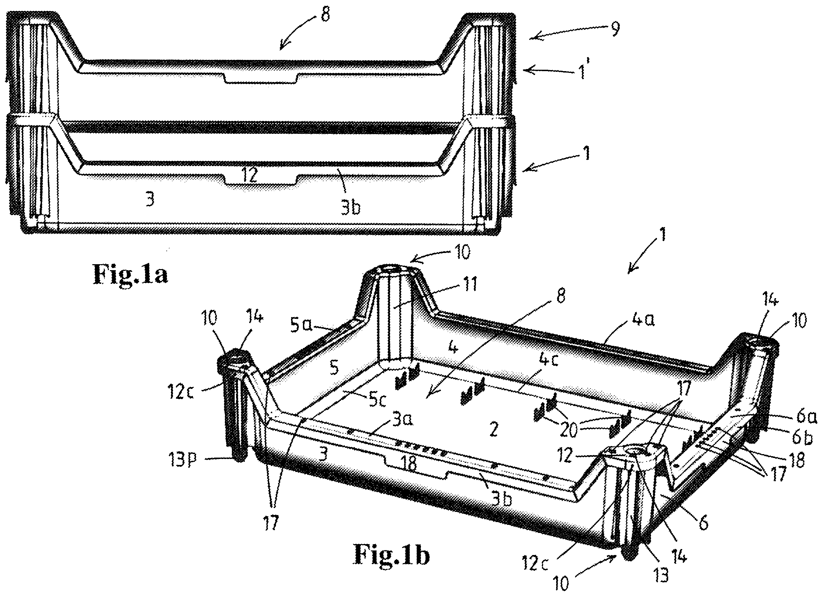

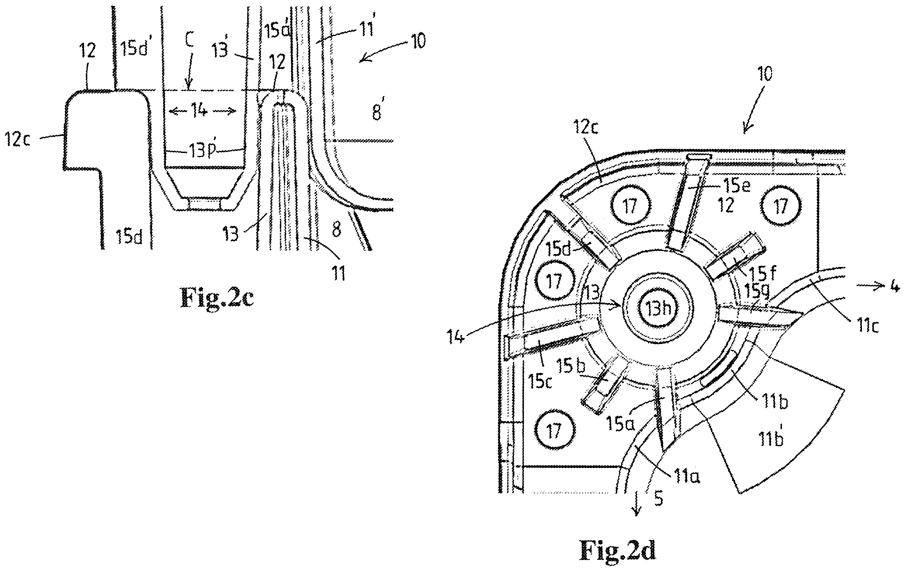

In figs. 1a -2d, an open topped, stackable, plastic container 1 of the invention is shown, with details of an inventive corner structure 10. In figs. 1a and 2c, two stacked containers 1 and 1' are shown, wherein same parts are given same reference numerals to which an apostrophe (') has been added.

The container is preferably a monolithically injection molded container, preferably made from PP. The container 1 is generally rectangular with a bottom 2 and first and second pairs of parallel upright walls 2, 3, 4, 5 joined to the bottom 2 and at corners via inventive corner structures 10, which will be discussed in detail below.

Here, at a bottom end, the upright walls 2, 3; 4, 5 join (and blend into) the bottom via curved sections 2c, 3c; 4c, 5c. A common wall thickness for this type of containers is 2-3,5 mm.

The dimensions of such a container are e.g. a length of 700-800 mm, a width of 500-600 mm and a height of 100-200 mm. With outer dimensions of length 79 × 59 × 19 cm, an containment space 8 of the container of over 2700 cm² is obtained.

Such a container is suitable for use in insect breeding/ rearing, e.g. to house egg-laying mother insects, possibly in combination with a spawn structure such as according to the second aspect not forming part of the invention. In addition, the containers can be used for hatching eggs, rearing baby-larvae and the process of rearing larvae into adult insects.

It is conceivable that the container is provided with spawn structure clamps 20, to be used in combination according to the second aspect not forming part of the invention. This will be further elucidated below. The container may also be suitable for accommodating a hatch structure, e.g. a hatch structure in which parts of the spawn structure of the second aspect onto which the eggs are laid are accommodated. Possibly, the bottom of the container is at least partly removed to allow eggs or larvae to drop from the spawn structure into another container.

In embodiments, e.g. when the container is used for housing mother insects, it is possible to remove at least a part of the bottom of the container, and replace it by a mesh, e.g. for the removal of excrements.

In fig. 1a, a stack 9 of two containers according to the invention is shown. Advantageously, a stack comprises at least twenty containers. Preferably, a few stacks are positioned on a pallet, e.g. 2-4 stacks. Advantageously, an insect breeding facility is provided with one or more climate housings, or climate areas, in which multiple stacks of containers are provided. The corner structure 10 according to the first aspect of the invention will be explained in relation to the corner structure 10 in fig. 1b between walls 4 and 5. In the shown embodiment, all four corner structures 10 have the same configuration.

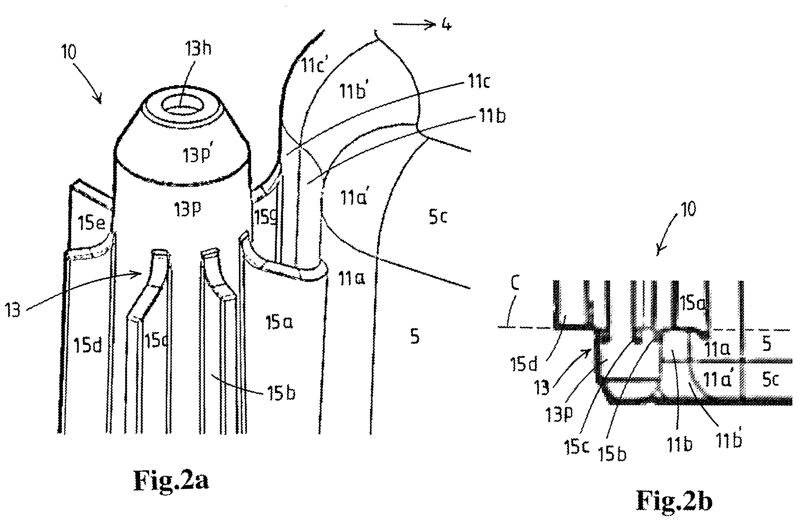

The corner structure 10 comprises a substantially diagonal and upright wall section 11 which, seen in top view, extends diagonally between adjoining upright walls 4,5 and blends into said upright walls to define the contour of a containment space 8 of the container. In particular, in the shown embodiment, as visible in fig. 1b and figs. 2a, 2b, and 2d, the upright wall section 11 comprises three portions: portion 11a adjacent wall 5, upright wall portion 11c adjacent wall 4, and upright wall portion 11b therebetween.

At a bottom end, the upright wall section 11 is joined to the bottom similar to the type of join of the upright walls to the bottom, via curved sections 11a1, 11b1, 11c1.

In the shown embodiment, a top wall 12 extends outward from an upper end of the diagonal and upright wall section, to extend essentially parallel to the bottom 2.

In the shown embodiment, the first and second pairs of parallel upright walls 2,3; 4,5 comprise a top flange 2a, 3a; 4a, 5a extending outward from an upper end of the upright walls. Here, the top flanges and the top walls 12 all extend horizontally, parallel to the bottom 2 of the container. Here, both the top flanges and the top walls 12 are provided with drainage holes 17, as visible in fig. 1b and in fig. 2d.

In the shown embodiment, the top flanges 2a, 3a; 4a, 5a are further provided with flange skirt walls 2b, 3b; 4b, 5b, depending downwards from the top flanges, essentially parallel to and at a distance from the first and second pairs of parallel upright walls, and wherein preferably at least one of the skirt walls is provided with a label 18, e.g. via in-mould labelling.

Here, also the corner structure 10 comprises a quarter-circular skirt wall 12c, depending downwards from an outward perimeter of the top wall 12 and at a distance from the diagonal and upright wall section.

According to the invention, a hollow tubular post 13 extends vertically downward from said top wall 12, said hollow tubular post 13 being spaced from said diagonal and upright wall section. Here the tubular post 13 is also spaced from the quarter-circular skirt wall 12c. The top wall has a hole 14 aligned with the hollow tubular post 13. Multiple, e.g. at least three, and in the shown embodiment 7 vertical stacking ribs 15a-15g are arranged circumferentially spaced about the hollow tubular post 13, each stacking rib having a vertical inner end joined to said hollow tubular post. The stacking ribs have an exemplary thickness of 2-4 mm.

At least one, here two of said stacking ribs 15a, 15g have vertical outer end joined to said diagonal and upright wall section 11a, 11c respectively. It is also conceivable that the vertical outer ends are joined to a wall of the first and second pairs of parallel upright walls. Furthermore, here part of vertical outer ends of stacking ribs 15c, 15d, 15e is joined to said quarter-circular skirt wall 12c. The vertical outer end of stacking ribs 15b, 15f is not joined to any other part.

Each of the stacking ribs has an upper end. Here, the upper ends of said stacking ribs 15a-15g join said top wall 12. Furthermore, each of the stacking ribs has a lower end. According to the first aspect of the invention, said lower ends are located in a common plane C. Here, the lower ends are slightly rounded, which is advantageous, as visible in fig. 2c, to match the hole 14 in which the protruding stacking pin portion is received when stacked.

The tubular post 13 has a protruding stacking pin portion 13p extending downward beyond said common plane of the stacking rib lower ends. In the shown embodiment, the protruding stacking pin portion 13p comprises a tapering end portion 13p'. Here, the bottom end of the stacking pin portion 13p is provided with a drainage hole 13h. As visible in particular in fig. 2c, in a stack the pin portion 13p' of an upper container 1' is received through the hole 14 in the top wall 12 of the corner structure of a lower container 1 onto which said upper container is stacked.

In the shown preferred embodiment, said stacking pin portion 13p does not protrude downward beyond the bottom 2 of the container and the lower ends of the stacking ribs in common plane C lie above the bottom so that - in a stack - the upper container nests in the lower container. In particular, the lower ends of the stacking ribs of the upper container, here ribs 15d' and 15a' are visible, rest onto the top wall 12 of the lower container, while the stacking pin portion 13p' is stacked through the hole 14 into the tubular post 13 of the lower container 1. Here, in a stack, the bottom 2' of the upper container is slightly below the level of the top walls of the corner structures of the lower container. Hence, there is an overlap between the containers 1 and 1'. In other words, the relative distance between the containers is negative.

The length of the stacking pin portion attributes to the stability of a stack of containers. An advantageous length is 30 mm, for containers having a height of 190mm, resulting in a stacking height of a container of about 160 mm.

In embodiments (not shown) with the stacking pin portion 13p protruding downward beyond the bottom 2 of the container, the lowermost container of a stack will rest on the stacking pin portions and not on its bottom. Furthermore, the relative distance between containers may be enlarged. The relative distance between containers is determined by the location of the common plane C of the lower ends of the stacking ribs.

In embodiments (not shown) with the bottom end of the stacking pin portion 13p at a distance above the bottom 2 of the container, the lowermost container of a stack will rest on the bottom and not on the stacking pin portions. Furthermore, the relative distance between containers may be very small, such that the containers overlap to a larger extent.

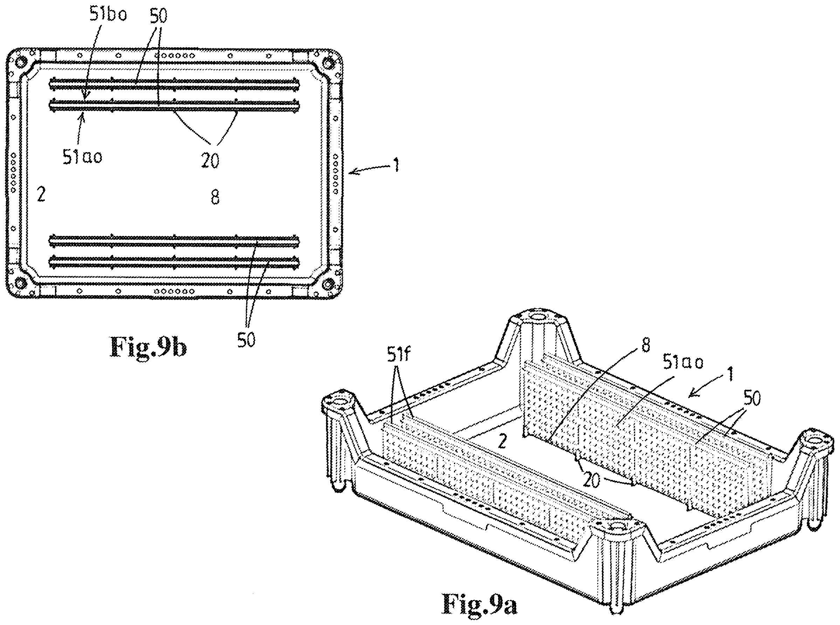

The second aspect, not forming part of the invention relates to the combination of an insect spawning container 1 and at least one spawn structure 50, an example of which is shown in figs 9a and 9b. The insect spawning container 1 here corresponds to the container 1 of the first aspect of the invention. Alternative container configurations are also conceivable, in particular stackable containers, as long as they are suitable to be used as an insect spawning container in an insect breeding facility for breeding insects of the type with crawling mother beetles having a protractable egg-laying tube, such as lesser mealworms or zophobas morios. The insect spawning container 1 comprises a bottom 2, first and second pairs 2,3; 4,5 of parallel upright walls and a containment space 8 for the mother beetles.

A spawn structure 50, here 4 spawn structures 50, is provided in combination with the container 1, for the mother insects to spawn their eggs. Here, the spawn structures 50 are provided in the longitudinal direction of the spawning container 1, at side parts of the container, leaving the central area between the spawn structures 50 open. In the shown configuration, the length of the spawn structure is between 70-100%, in particular 80-90%, of that of the insect spawning container. The width of a spawn structure is relatively small, in the order of 1-5 % of the width of the container, in particular in the order of 8-20 mm, in particular 10-15 mm.

In the shown embodiment, as visible in fig. 1b, fig. 9a and 9b, the bottom 2 of the container is possibly provided with spawn structure clamps 20, to receive a spawn structure in the spawning configuration and allow the spawn structure to be connected to the container 2. Preferably, this is a spawn structure according to the second aspect of the invention.

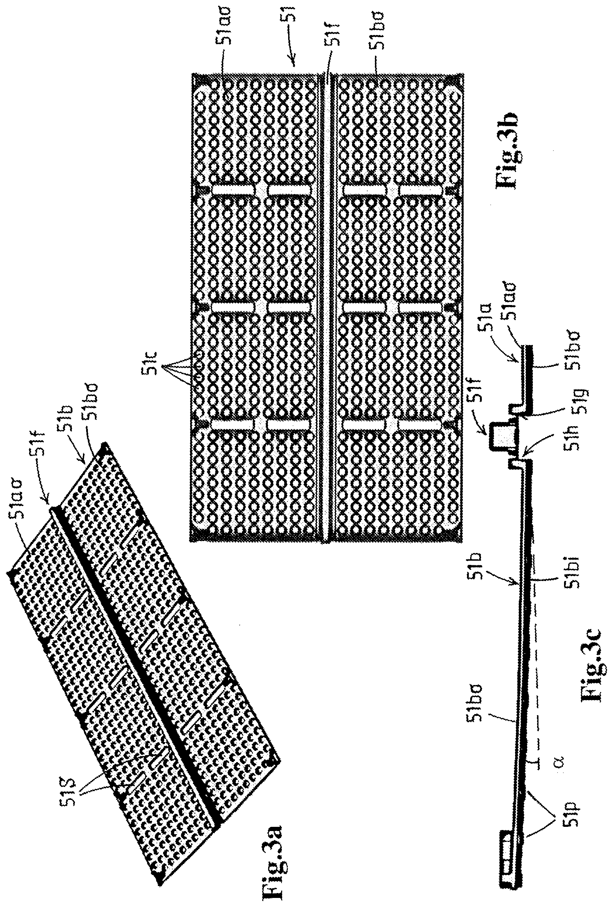

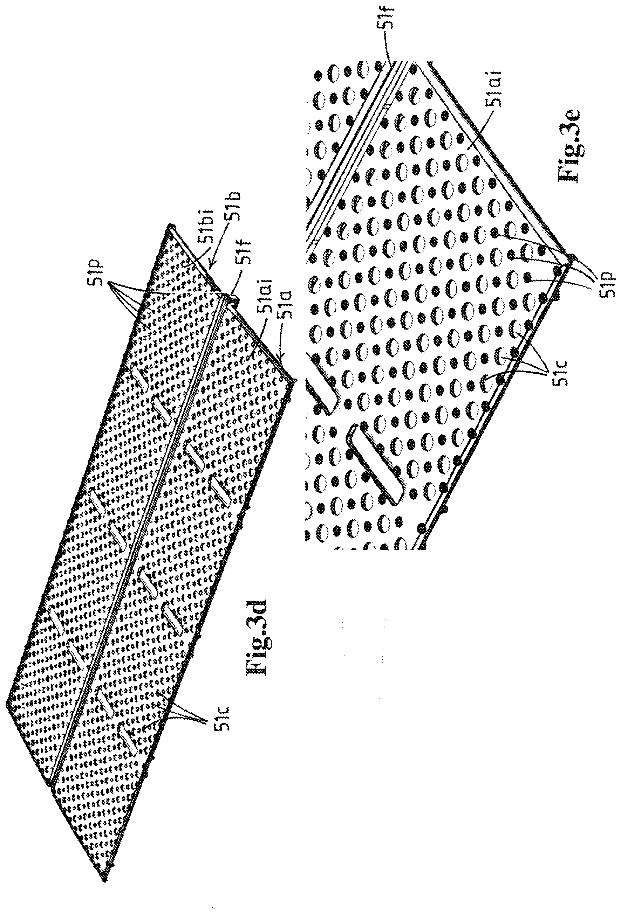

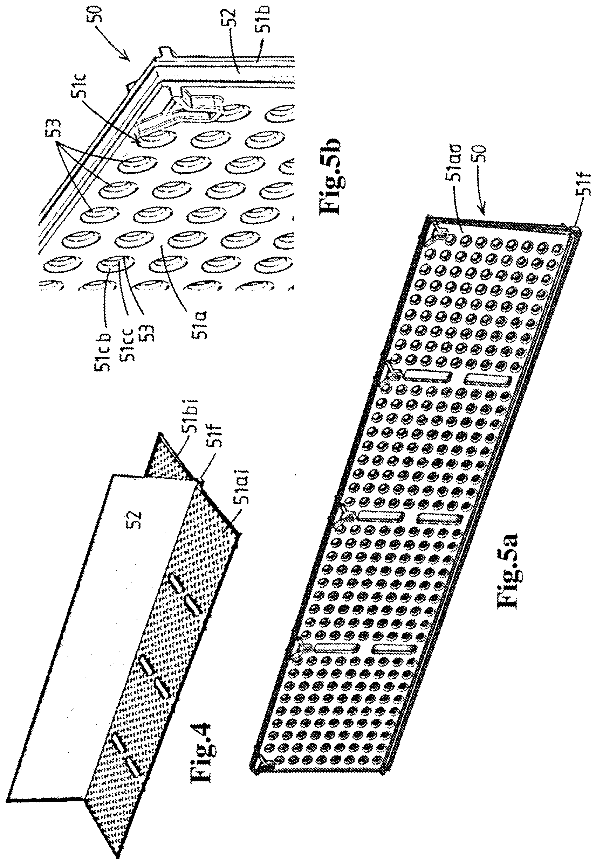

The spawn structure comprises a foldable member 51 with perforations or cut-aways 51c, as shown in figs. 3a- 3e, and a rigid plate 52, visible in fig. 4. The foldable member 51 and the rigid plate 52 are preferably monolithically injection molded plastic products.

In figs. 3a-3e the foldable member 51 of the spawn structure according to the second aspect of the invention is shown in detail, without the rigid plate 52 of the spawn structure 50.

According to the second aspect this foldable member 51 comprises a fold section 51f provided centrally between, and via hinges 51g, 51h connected to, a perforated left-hand section 51a and a perforated right-hand section 51b. The left-hand section 51a and right-hand section 51b are embodied as plates, and are provided with perforations 51c. In the shown configuration, the foldable member 51 comprises 8 segments, here each provided with 80-81 perforations. Each section 51a, 51b comprises an outer face 51ao, 51bo and an inner face 51ai, 51bi, respectively.

According to the second aspect the foldable member and the rigid plate are movable with respect to each other between an open configuration and a closed spawning configuration. The mutual distance between the foldable member and rigid plate is increased and decreased during this movement, in particular the distance between the inner faces 51ai, 51bi.

In figs. 5a, 5b and 9a, 9b the spawn structure 50 is shown in a closed spawning configuration. In the spawning configuration, the foldable member 51 is folded such that the left-hand section 51a and the right-hand section 51b are parallel to each other. The rigid plate 52 is sandwitched between the sections 51a, 51b. A multitude of crevices 53 is created between the rigid plate 52 and inner faces 51ai, 51bi of the folded member. These crevices 53 have dimensions tuned to the egg-laying tube of the mother beetles. For breeding lesser mealworms the width of the crevices is 0,2-1,2 mm, while for breeding zophobas morios the width of the crevices is 0,2-1,8 mm. The depth of the crevices may vary between 0,5-10,0 mm.

In the spawning configuration, as visible in figs. 9a and 9b, the spawn structures 50 are positioned in the container, here adjacent the bottom 2. The outer faces 51ao, 51bo of the folded member 51 form two climbing faces which are positioned adjacent the bottom of the container. An advantage of the foldable member is that crevices are created at both sides of the spawn structure.

The spawn structure in the closed spawning configuration is positioned in the container such that the mother beetles are able to crawl from the bottom onto and up along the climbing faces of the spawn structure into the crevices, e.g. via the spawn structure clamps 20.

In an open configuration, as visible in fig. 4, the foldable member 51 is folded open, and the distance between the foldable member 51 and rigid plate 52 is increased, allowing harvesting of the eggs, and/ or cleaning of the spawn structure.

The configuration of the spawn structure according to the second aspect allows efficient handling of the spawn structure, e.g. to switch between the closed and the open configuration. An increase in efficiency will may attribute to an increased yield, in particular during scaling up the breeding of insects.

Furthermore, an increased yield is obtained by allowing the opening of the spawn structure by a sideways movement of the foldable member 51, in particular of the left-hand part 51a and the right-hand part 51b, similar to the opening of a book. This side-ways opening diminishes the risk of damaging the eggs, as no transversal relative sliding movement of the foldable member and rigid plate of the spawn structure is possible.

Also attributing to the prevention of relative sliding is providing the fold section 51f with an elongated slit seat 51fs having a length corresponding to the length of the rigid plate, a width essentially corresponding to the thickness of the rigid plate, and a depth to accommodate the rigid plate.

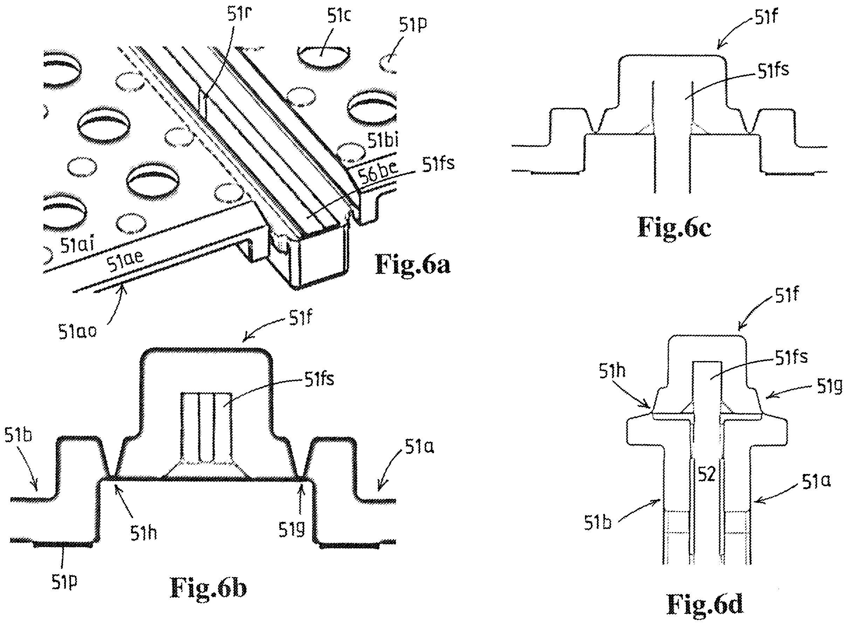

This is shown in more detail in figs. 6a-6d. In particular, in fig. 6a the 'empty' slit 51fs without the rigid plate 52 is shown. Also, an optionally provided rib 51r in the slit 51fs is visible, which may be provided to attribute to fixation of the rigid plate 52.

In fig. 6d, the rigid plate 52 is positioned into the slit seat 51fs.

The yield correlates with the number of crevices 53 having the correct dimensions.

In particular in fig. 5b, a detail of an advantageous type perforation is visible. The perforations 51c are circular, and here provided with a bevelled part 51cb, such that the perforation 51c comprises a relatively large diameter adjacent the outer surface 51ao of the foldable plate, tapering to a smaller diameter adjacent a cylindrical part 51cc of the perforation, which ends at the inner surface 51ai of the foldable plate 51. The crevices 53 are formed between the end of the cylindrical part 51cc and the rigid plate 52.

Advantageously, in the configuration as shown as can be derived in particular from fig. 4, the foldable member 51 has a length in the direction of the fold section 51f, essentially corresponding to the length of the rigid plate 52, and wherein the width of the left-hand and right-hand sections essentially correspond to the width of the rigid plate. As a result, no additional crevices or clear spaces are created between the foldable member and rigid plate, other than the envisaged crevices.

To obtain crevices having dimensions tuned to the egg-laying tube of the mother beetles, advantageously the inner faces 51ai, 51bi of the foldable member are provided with protrusions 51p, in particular visible in fig. 3e. E.g. protrusions of 0,3 - 0,4mm high are suitable to create crevices of the desired dimensions.

In view of the importance of the dimensions of the crevices, it is important to accurately control the mutual distance between the foldable member and the rigid plate in the closed spawning configuration. The provision of protrusions 51p ensures a minimum distance between the parts of the spawn structure. On the other hand, measures may be contemplated to reduce the risk of having a too large mutual distance.

E.g. in fig. 3a, elongated savings 51s are visible between the perforated sections. These are provided to prevent bulging out of the left- and right-hand sections.

Another measure to control the dimensions of the crevices is to provide a pre-stress to at least one of the hinges 51g, 51h between the left-hand and right-hand sections 51a, 51b and the fold section 51f, opposite the folding direction.

In fig. 3c, the pre-stress is visible as a slight angle α of 1-1,5° between the right-hand section 51b and the horizontal, while here the left-hand section 51 does not include an angle with the horizontal.

In fig. 6a, it is visible that sides 51ae, 51be of the left-hand section 51a and right-hand section 51b respectively of the foldable member 51, adjacent the fold section 51f, taper towards the outer faces 51ao, 51bo respectively. By allowing the ends to taper towards the outer faces, in the spawning configuration an opening is created, between the parallel left-hand section and the right-hand section on the one hand, and the rigid plate on the other hand. This opening facilitates the increasing of the distance between the foldable member and rigid plate of the spawn structure to the open configuration. Possibly, the opening allow the use of an opening tool to increase the mutual distance.
wherein the ends of the inner faces of the foldable member opposite the fold section and/or the sides of the foldable member adjacent the fold section taper towards the outer faces.

Yet another optional measure to control the dimensions of the crevices is to ensure a proper closing of the spawn structure in the closed spawning configuration. For example, a clip is provided to close the sandwich of left-hand section 51a, rigid plate 52 and right-hand section 51b opposite the fold section 51f.

In the shown embodiments, the spawn structure is positioned in the crate via spawn structure clamps 20 provided at the bottom of the insect spawning container 1. The design of these clamps 20 attributes to a proper closing of the sandwiched spawn structure 50.

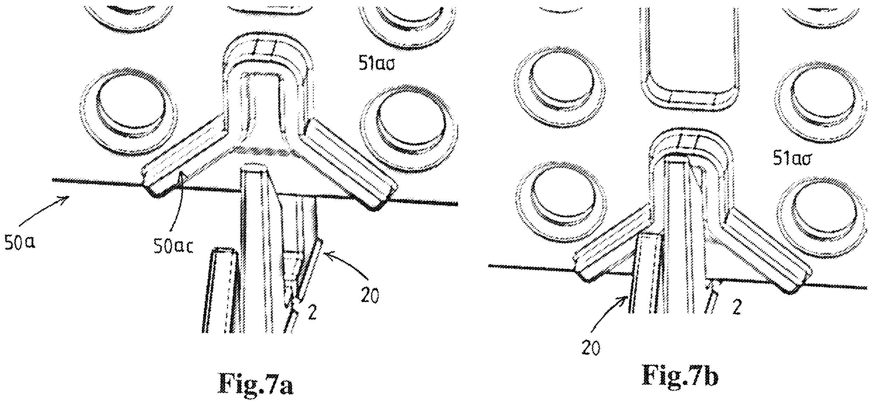

In figs. 7a-8d, the spawn structure clamp 20 is shown in further detail. The bottom 2 of the insect spawning container 1 is provided with spawn structure clamps 20 to receive the spawn structure 50 in the spawning configuration, and allow the spawn structure to be connected to the container.

In the shown embodiment, the spawn structure clamp 20 comprises opposed protruding fingers 20a, 20b, protruding from the bottom 2 of the insect spawning container, between which a spawn structure receiving groove 20c is provided. The configuration of the opposed fingers is such that the bottom of the groove between the fingers is provided at a distance from the bottom of the spawning container, e.g. 2-4, preferably 3 mm.

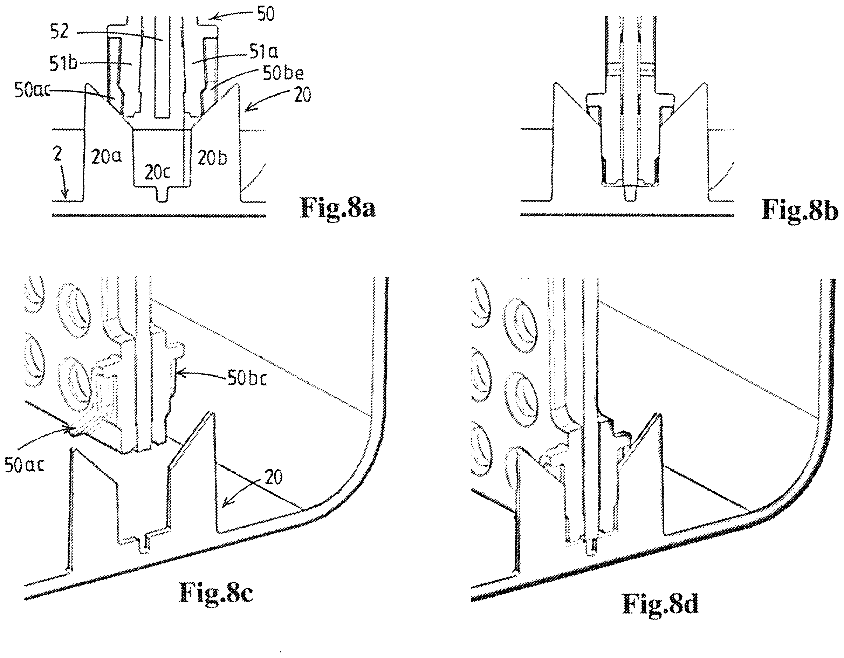

The fingers have tapering ends, to provide a guide surface 20g for the spawn structure. This is I particular visible in figs. 8a and 8b, in which the entrance of the spawn structure 50, and the closing to the closed spawning configuration is elucidated.

To define the position of the spawn structure 50 with respect to the container 1, advantageously the ends of the outer faces 50ao, 50bo of the foldable member opposite the fold section are provided with complementary container connectors 50ac, 50bc. The complementary container connectors 50ac, 50bc allow the spawn structure 50 to be connected to the container 1 in the spawning configuration. Here, the configuration of the spawn structure clamps 20 and the complementary container connectors 50ac, 50bc is such that by connecting the spawn structure to the container, the position of the spawn structure in the container is defined. Possible container connectors are snap-fitments, protruding from the outer faces.

## Claims

1. Open topped, stackable, molded plastic container (1), which is generally rectangular with a bottom (2) and first and second pairs (2,3; 4,5) of parallel upright walls joined to the bottom and at corners via corner structures (10), wherein each corner structure comprises a substantially diagonal and upright wall section (11) which, seen in top view, extends diagonally between adjoining upright walls and blends into said upright walls to define the contour of a containment space (8) of the container; **characterized in that** each corner structure further comprises:
- a top wall (12) extending outward from an upper end of the diagonal and upright wall section; and
- a hollow tubular post (13) extending vertically downward from said top wall (12), said hollow tubular post being spaced from said diagonal and upright wall section (11), wherein the top wall has a hole (14) aligned with the hollow tubular post (13);
- wherein multiple, e.g. at least three, vertical stacking ribs (15a-15g) are arranged circumferentially spaced about the hollow tubular post, each stacking rib having a vertical inner end joined to said hollow tubular post,
∘ wherein at least one, preferably two, of said stacking ribs (15a, 15g) have a vertical outer end joined to said diagonal and upright wall section (11) and/or to said adjoining upright wall section; and
∘ wherein each of said stacking ribs has a lower end, said lower ends being located in a common plane (C);
wherein said tubular post has a protruding stacking pin portion (13p) extending downward beyond said common plane of the stacking rib lower ends; such that - in a stack - the pin portion of an upper container is received through the hole in the top wall of the corner structure of a lower container onto which said upper container is stacked.

2. Container according to claim 1, wherein said stacking pin portion does not protrude downward beyond the bottom of the container and the lower ends of the stacking ribs lie above the bottom so that - in a stack - the upper container nests in the lower container.

3. Container according to claim 1 or 2, wherein each stacking rib has an upper end joining said top wall 12.

4. Container according to any of claims 1-3, wherein the corner structure further comprises a quarter-circular skirt wall (12c), extending downwards from an outward perimeter of the top wall; and wherein preferably part of a vertical outer end of at least one stacking rib (15c, 15d, 15e) is joined to said quarter-circular skirt wall.

5. Container according to any of claims 1-4, wherein a top flange (2a, 3a; 4a, 5a) extends outward from an upper end of each upright wall (2,3; 4,5), wherein preferably the top flanges and/ or the top walls are provided with drainage holes (17).

6. Container according to claim 5, wherein flange skirt walls (2b, 3b; 4b, 5b) depend downwards from the top flanges, essentially parallel to and at a distance from the respective upright walls, and wherein preferably at least one of the skirt walls is provided with a label (18).

7. Container according to any of claims 1-6, adapted for use in an insect breeding facility for breeding insects of the type with crawling mother beetles having a protractable egg-laying tube, such as lesser mealworms or zophobas morios; adapted to be used in combination with at least one spawn structure (50) in which the mother insects will spawn their eggs, wherein the bottom of the container is provided with spawn structure clamps to receive the spawn structure and allow the spawn structure to be connected to the container.

8. Use of a container according to one or more of the preceding claims in an insect breeding facility.

9. Stack (2) comprising at least 15, preferably at least 20 containers according to one or more of the preceding claims 1-7.

10. Climate housing of an insect breeding facility, comprising multiple stacks according to claim 9.

## Patentansprüche

1. Oben offener, stapelbarer, geformter Kunststoffbehälter (1), der im Allgemeinen rechteckig mit einem Boden (2) und ersten und zweiten Paaren (2, 3; 4, 5) von parallelen aufrechten Wänden ist, die mit dem Boden, und an Ecken über Eckenstrukturen (10) verbunden sind, wobei jede Eckenstruktur einen im Wesentlichen diagonalen und aufrechten Wandabschnitt (11) umfasst, der sich in Draufsicht gesehen diagonal zwischen angrenzenden aufrechten Wänden erstreckt, und sich in die aufrechten Wände einfügt, um die Kontur eines Eingrenzungsraumes (8) des Behälters zu definieren;
**dadurch gekennzeichnet, dass** jede Eckenstruktur weiter umfasst:
- eine obere Wand (12), die sich von einem oberen Ende des diagonalen und aufrechten Wandabschnitts nach außen erstreckt; und
- einen hohlen rohrförmigen Ständer (13), der sich von der oberen Wand (12) vertikal nach unten erstreckt, wobei der hohle rohrförmige Ständer von dem diagonalen und aufrechten Wandabschnitt (11) beabstandet ist, wobei die obere Wand ein Loch (14) aufweist, das mit dem hohlen rohrförmigen Ständer (13) fluchtet;
- wobei mehrere, z.B. mindestens drei vertikale Stapelrippen (15a-15g) umfänglich beabstandet um den hohlen rohrförmigen Ständer herum angeordnet sind, wobei jede Stapelrippe ein vertikales inneres Ende aufweist, das mit dem hohlen rohrförmigen Ständer verbunden ist,
° wobei mindestens eine, vorzugsweise zwei der Stapelrippen (15a-15g) ein vertikales äußeres Ende aufweisen, das mit dem diagonalen und aufrechten Wandabschnitt (11) und/oder mit dem angrenzenden aufrechten Wandabschnitt verbunden ist; und
° wobei jede der Stapelrippen ein unteres Ende aufweist, wobei sich die unteren Enden in einer gemeinsamen Ebene (C) befinden;
wobei der rohrförmige Ständer einen vorspringenden Stapelstiftabschnitt (13p) aufweist, der sich nach unten über die gemeinsame Ebene der unteren Enden der Stapelrippe hinaus erstreckt; sodass - in einem Stapel - der Stiftabschnitt eines oberen Behälters durch das Loch in der oberen Wand der Eckenstruktur eines unteren Behälters, auf den der obere Behälter gestapelt ist, aufgenommen wird.

2. Behälter nach Anspruch 1, wobei der Stapelstiftabschnitt nicht nach unten über den Boden des Behälters hinaus übersteht, und die unteren Enden der Stapelrippen über dem Boden liegen, sodass sich der obere Behälter - in einem Stapel - in den unteren Behälter einnistet.

3. Behälter nach Anspruch 1 oder 2, wobei jede Stapelrippe ein oberes Ende aufweist, das mit der oberen Wand (12) verbunden ist.

4. Behälter nach einem der Ansprüche 1-3, wobei die Eckenstruktur weiter eine viertelkreisförmige Schaftwand (12c) umfasst, die sich von einem Außenumfang der oberen Wand nach unten erstreckt; und wobei vorzugsweise ein Teil eines vertikalen äußeren Endes mindestens einer Stapelrippe (15c, 15d, 15e) mit der viertelkreisförmigen Schaftwand verbunden ist.

5. Behälter nach einem der Ansprüche 1-4, wobei sich ein oberer Flansch (2a, 3a; 4a, 5a) von einem oberen Ende einer jeden aufrechten Wand (2, 3; 4, 5) nach außen erstreckt, wobei die oberen Flansche und/oder die oberen Wände vorzugsweise mit Drainagelöchern (17) bereitgestellt sind.

6. Behälter nach Anspruch 5, wobei Flansch-Schaftwände (2b, 3b; 4b, 5b) von den oberen Flanschen, im Wesentlichen parallel zu, und in einem Abstand von den jeweiligen aufrechten Wänden nach unten herabhängen, und wobei mindestens eine der Schaftwände vorzugsweise mit einem Etikett (18) bereitgestellt ist.

7. Behälter nach einem der Ansprüche 1-6, zur Verwendung in einer Insektenzuchtanlage zum Züchten von Insekten in der Art mit krabbelnden Mutterkäfern, die ein verlängerbares Eiablagerohr aufweisen, wie Buffalowürmer oder Schwarzlarvenkäfer; angepasst, um in Kombination mit mindestens einer Laichstruktur (50) verwendet zu werden, in der die Mutterinsekten ihre Eier ablaichen, wobei der Boden des Behälters mit Laichstrukturklemmen bereitgestellt ist, um die Laichstruktur aufzunehmen, und es der Laichstruktur zu ermöglichen, mit dem Behälter verbunden zu werden.

8. Verwendung eines Behälters nach einem oder mehreren der vorstehenden Ansprüche in einer Insektenzuchtanlage.

9. Stapel (2), der mindestens 15, vorzugsweise mindestens 20 Behälter nach einem oder mehreren der vorstehenden Ansprüche 1-7 umfasst.

10. Klimagehäuse einer Insektenzuchtanlage, die mehrere Stapel nach Anspruch 9 umfasst.

## Revendications

1. Contenant en plastique moulé, empilable, à partie supérieure ouverte (1), qui est généralement rectangulaire avec un fond (2) et des première et deuxième paires (2, 3 ; 4, 5) de parois verticales parallèles raccordées au fond et aux coins par l'intermédiaire de structures de coin (10), dans lequel chaque structure de coin comprend une section de paroi sensiblement diagonale et verticale (11) qui, vue selon une vue de dessus, s'étend en diagonale entre des parois verticales attenantes et se mélange auxdites parois verticales pour définir le contour d'un espace de confinement (8) du contenant ; **caractérisé en ce que** chaque structure de coin comprend en outre :
- une paroi supérieure (12) s'étendant vers l'extérieur depuis une extrémité supérieure de la section de paroi diagonale et verticale ; et
- un montant tubulaire creux (13) s'étendant verticalement vers le bas depuis ladite paroi supérieure (12), ledit montant tubulaire creux étant espacé de ladite section de paroi diagonale et verticale (11), dans lequel la paroi supérieure présente un trou (14) aligné sur le montant tubulaire creux (13) ;
- dans lequel de multiples, par exemple au moins trois, nervures d'empilement verticales (15a-15g) sont agencées de manière circonférentiellement espacée autour du montant tubulaire creux, chaque nervure d'empilement ayant une extrémité intérieure verticale raccordée audit montant tubulaire creux,
o dans lequel au moins une, de préférence deux, desdites nervures d'empilement (15a, 15g) comporte une extrémité extérieure verticale raccordée à ladite section de paroi diagonale et verticale (11) et/ou à ladite section de paroi verticale attenante ; et
o dans lequel chacune desdites nervures d'empilement comporte une extrémité inférieure, lesdites extrémités inférieures étant situées dans un plan commun (C) ;
dans lequel ledit montant tubulaire a une portion broche d'empilement saillante (13p) s'étendant vers le bas au-delà dudit plan commun des extrémités inférieures de nervure d'empilement ; de telle sorte que - dans une pile - la portion broche d'un contenant supérieur est reçue à travers le trou de la paroi supérieure de la structure de coin d'un contenant inférieur sur lequel est empilé ledit contenant supérieur.

2. Contenant selon la revendication 1, dans lequel ladite portion broche d'empilement ne fait pas saillie vers le bas au-delà du fond du contenant et les extrémités inférieures des nervures d'empilement reposent au-dessus du fond de telle sorte que - dans une pile - le contenant supérieur s'emboîte dans le contenant inférieur.

3. Contenant selon la revendication 1 ou 2, dans lequel chaque nervure d'empilement a une extrémité supérieure se raccordant à ladite paroi supérieure 12.

4. Contenant selon l'une quelconque des revendications 1-3, dans lequel la structure de coin comprend en outre une paroi de jupe en quart de cercle (12c), s'étendant vers le bas depuis une périphérie extérieure de la paroi supérieure ; et dans lequel de préférence une partie d'une extrémité extérieure verticale d'au moins une nervure d'empilement (15c, 15d, 15e) est raccordée à ladite paroi de jupe en quart de cercle.

5. Contenant selon l'une quelconque des revendications 1-4, dans lequel un rebord supérieur (2a, 3a ; 4a, 5a) s'étend vers l'extérieur depuis une extrémité supérieure de chaque paroi verticale (2, 3 ; 4, 5), dans lequel de préférence les rebords supérieurs et/ou les parois supérieures sont dotés de trous de drainage (17).

6. Contenant selon la revendication 5, dans lequel des parois de jupe de rebord (2b, 3b ; 4b, 5b) pendent vers le bas depuis les rebords supérieurs, essentiellement parallèlement aux parois verticales respectives et à une distance de ces dernières, et dans lequel de préférence au moins une des parois de jupe est dotée d'une étiquette (18).

7. Contenant selon l'une quelconque des revendications 1-6, adapté pour être utilisé dans une installation de reproduction d'insectes destinée à la reproduction d'insectes du type coléoptères mères rampants ayant un tube ovipositeur protractile, tels que les petits ténébrions mats ou les zophobas morios ; adapté pour être utilisé en combinaison avec au moins une structure de frai (50) dans laquelle les insectes mères pondront leurs œufs, dans lequel le fond du contenant est doté de brides de structure de frai pour recevoir la structure de frai et permettre à la structure de frai d'être reliée au contenant.

8. Utilisation d'un contenant selon une ou plusieurs des revendications précédentes dans une installation de reproduction d'insectes.

9. Pile (2) comprenant au moins 15, de préférence au moins 20 contenants selon une ou plusieurs des revendications précédentes 1-7.

10. Logement climatique d'une installation de reproduction d'insectes, comprenant de multiples piles selon la revendication 9.
